# EUROPEAN PATENT SPECIFICATION

(11) **EP 2 185 581 B1**
(45) Date of publication and mention of the grant of the patent: **02.09.2015**
(21) Application number: 08826168.0
(22) Date of filing: 07.07.2008
(51) Int. Cl.: C07K 14/47, A61K 38/17, A61K 39/395, A61K 38/39

(54) **METHODS AND COMPOSITIONS USEFUL IN THE TREATMENT OF MUCOSITIS**
VERFAHREN UND ZUSAMMENSETZUNGEN ZUR BEHANDLUNG VON MUKOSITIS
PROCÉDÉES ET COMPOSITIONS UTILES DANS LE TRAITEMENT DE LA MUCOSITE

(30) Priority: 06.07.2007 US 958634 P; 20.07.2007 US 961343 P; 27.06.2008 US 215700
(43) Date of publication of application: 19.05.2010
(73) Proprietor: Promedior, Inc., Malvern, PA 19355 (US)
(72) Inventor: HESSON, David, Paul, Malvern, PA 19355 (US); KRAMER, Michael, Scott, Harleysville, PA 19438 (US)
(74) Representative: Vossius & Partner Patentanwälte Rechtsanwälte mbB
(86) International application number: PCT/US2008/008340
(87) International publication number: WO 2009/009034

(56) References cited:
- WO-A-92/21364
- WO-A-95/05394
- WO-A-2007/047796
- WO-A-2008/070117
- GARDEN AS, CHAMBERS MS: "Head and neck radiation and mucositis" CURR OPIN SUPPORT PALLIAT CARE, vol. 1, no. 1, April 2007 (2007-04), XP009109809
- KIVELÄ-RAJAMÄKI MJ ET AL: "Laminin-5-gamma-2-chain and collagenase-2 (MMP-8) in human peri-implant sulcular fluid" CLIN. ORAL IMPL. RES., vol. 14, 2003, pages 158-165, XP002507773
- AIBA S, TAGAMI H: "immunoglobulin-producing cells in plasma cell orificial" JOURNAL OF CUTANEOUS PATHOLOGY, vol. 16, no. 4, 1989, pages 207-210, XP002507774
- PILLING D, ROIFE D, WANG M, RONKAINEN SD, CRAWFORD JR, TRAVIS EL, GOMER RH: "Reduction of bleomycin-induced pulmonary fibrosis by serum amyloid P" J IMMUNOL, vol. 179, no. 6, 15 September 2007 (2007-09-15), pages 4035-4044, XP002507775

## Description

### BACKGROUND OF THE INVENTION

Mucositis is a condition characterized by swelling, irritation, and discomfort of mucosal linings such as those of the gastrointestinal tract and the oral and oralpharyngeal cavities, and can result in mouth and throat sores, diarrhea, abdominal cramping and tenderness, and rectal ulcerations. This condition occurs in approximately half of all cancer patients undergoing therapy, and is a common side effect of cancer treatments involving radiation and/or chemotherapy. The goal of these approaches to cancer treatment is to kill rapidly dividing cancer cells but, unfortunately, other rapidly dividing cells are killed by the treatment as well, including cells that line regions such as the gastrointestinal tract, leading to mucositis.

The incidence of mucositis, as well as its severity, depends on factors such as the type and duration of the cancer treatment. Mucositis occurs, for example, in virtually all patients who are treated by irradiation of the head and neck. It is also highly prevalent in patients treated with high dose chemotherapy and/or irradiation for the purpose of myeloablation, such as in preparation for stem cell or bone marrow transplantation.

Mucositis adversely impacts the quality of life of cancer patients in several ways. For example, the mouth and throat sores of mucositis can cause significant pain and make it difficult to eat, drink, and even take oral medication. Mucositis is also accompanied by a severe risk of infection, as it can lead to a breach in the otherwise protective linings of the oral mucosa and gastrointestinal tract, which are colonized by a vast array of microorganisms. Gut toxicity is a major limiting factor in radiation and chemotherapy treatment regimes. Further, efforts to counter the discomforts of mucositis can lead to disruptions in cancer treatment, alterations in treatment dosages, or shifting to different modes of treatment. Severe mucositis can also lead to the need for parenteral nutrition or hospitalization. The development of effective approaches to preventing and treating mucositis is therefore important for improving the care of cancer patients.

Alimentary mucositis refers to a form of mucosal barrier injury to the alimentary tract. Alimentary mucositis may occur at a part or multiple parts of the alimentary tract, from mouth to anus, via, e.g., esophagus, stomach, small intestine, colon, and rectum. Non-limiting examples of alimentary mucositis are oral mucositis, esophagitis, stomatitis, enteritis, and proctitis. See, e.g., Blijlevens et al., Bone Marrow Transplant 25:1269-1278 (2000); and Keefe et al., Seminars in Oncology 20:38-47 (2004).

Alimentary mucositis are generally caused by one or more insults, most commonly by a chemical(s) or radiation, or a combination thereof. Radiation may be a result of, e.g., radiation therapy, accidental radiation exposure, and radiation exposure from a terrorist attack. See e.g., Moulder, Int. J. Radiat. Biol. 80:3-10 (2004). Chemical insults are commonly from chemotherapy.

### SUMMARY OF THE INVENTION

In part, the disclosure demonstrates that SAP polypeptides are useful in a treatment for mucositis. One aspect of the invention provides methods for treating, preventing or reducing the severity of mucositis in a patient by administering a therapeutically effective amount of an SAP agonist. The administration of an SAP polypeptide may delay the development of mucositis, reduce the number of days a patient is afflicted with mucositis, and/or reduce the severity of mucositis.

The application provides methods for treating patients afflicted with mucositis, as well as patients at risk of developing mucositis. Cancer therapy with radiation the, chemotherapy, or a combination thereof are treatments associated with a high risk of mucositis related side effects. The administration of SAP polypeptides may commence prior, concurrently, or after treatment with radiation therapy or chemotherapy. In some embodiments, patients are afflicted with cancer. In some embodiments, patients are afflicted with lung, ovarian, prostate, lymphoma or gastrointestinal cancer. In some embodiments, patients are afflicted with head and neck cancer or cancer requiring a bone marrow transplant, such as myeloablation therapy.

The application provides methods for treating or preventing a number of related disorders. Methods of the invention are useful for treating oral, esophageal, and gastrointestinal mucositis, as well as gastric and duodenal ulcers, or erosions of the stomach and esophagus.

The application provides SAP polypeptides useful in the methods of the invention. SAP polypeptides may increase or mimic SAP signaling or increase SAP activity.

### BRIEF DESCRIPTION OF THE DRAWINGS

**Figure 1** **(A-C)** depicts hematoxylin and eosin staining from a rat model of radiation-induced enteropathy. Staining of intestines from non-irradiated control (Fig1A), irradiatated vehicle-treated (Fig1B) and hSAP-treated rats (Fig1C).
**Figure 2** depicts mucosal surface area two weeks after radiation treatment in hSAP-treated and in irradiated vehicle treated rats.
**Figure 3** depicts subserosal thickness two weeks after radiation treatment in hSAP-treated and in irradiated vehicle treated rats.
**Figure 4** depicts the radiation injury score two weeks after radiation treatment in hSAP-treated and in irradiated vehicle treated rats.
**Figure 5** depicts type-III collagen immunoreactivity two weeks after radiation treatment in hSAP-treated and in irradiated vehicle treated rats.
**Figure 6** depicts the mean blinded mucositis scores in a hamster cheek pouch model of radiation-induced oral mucositis.
**Figure 7** depicts the mean blinded mucositis scores in hamster cheek pouch model of radition-induced oral mucositis.
**Figure 8** depicts depicts the mean blinded mucositis scores in hamster cheek pouch model of radition-induced oral mucositis.
**Figure 9** depicts the amino acid sequence alignment of human (SEQ ID NO: 1, amino acids 20-223 of Genbank Accession No. NP_001630), Gallus gallus (SEQ ID NO: 2, amino acids 20-227 of Genbank Accession No. NP_001034653), Bos taurus (SEQ ID NO: 3, amino acids 20-224 of Genbank Accession No. AAI02624), and Cricetulus migratorius (SEQ ID NO: 4, amino acids 20-223 of Genbank Accession No. AAB28726) serum amyloid P polypeptides (signal sequence not depicted). Amino acids identical to the human SAP are shaded.

### DETAILED DESCRIPTION OF THE INVENTION

### Overview

One aspect of the present invention relates to the surprising discovery that serum amyloid P (SAP) demonstrates a therapeutic affect in the treatment of mucositis.

Serum amyloid P ("SAP") is a naturally-occurring serum protein in mammals composed of five identical subunits or protomers which are non-covalently associated in a disc-like molecule. SAP is a 125,000 Dalton pentameric glycoprotein composed of five, non-covalently linked, 25,000 Dalton protomers. SAP belongs to the pentraxin superfamily of proteins, characterized by this cyclic pentameric structure. The classical short pentraxins include SAP as well as C-reactive protein. (Osmand, A. P., et al., Proc. Nat. Acad. Sci., 74:739-743 (1977)) It is synthesized in the liver and the physiological half-life of human SAP is 24 hours. The sequence of the human SAP subunit is depicted in SEQ ID NO:1 (amino acids 20-223 of Genbank Accession No. NP_001630, signal sequence not depicted).

### Definitions

As used herein, the terms "treatment", "treating", and the like, refer to obtaining a desired pharmacologic and/or physiologic effect. The effect may be prophylactic in terms of completely or partially preventing a disorder or symptom thereof and/or may be therapeutic in terms of a partial or complete cure for a mucositis disorder and/or adverse affect attributable to the disorder. "Treatment", as used herein, covers any treatment of a disease in a mammal, particularly in a human, and includes: (a) increasing-survival time; (b) decreasing the risk of death due to the disease; (c) preventing the disease from occurring in a subject which may be predisposed to the disease but has not yet been diagnosed as having it; (d) inhibiting the disease, i.e., arresting its development (e.g., reducing the rate of disease progression); and (e) relieving the disease, i.e., causing regression of the disease.

As used herein, a therapeutic that "prevents" a disorder or condition is a compound that, in a statistical sample, reduces the occurrence of the disorder or condition in the treated sample relative to an untreated control sample, or delays the onset or reduces the severity of one or more symptoms of the disorder or condition relative to the untreated control sample.

As used herein the terms "subject" and "patient" refer to animals including mammals, in particular humans. The term "mammal" includes primates, domesticated animals including dogs, cats, sheep, cattle, goats, pigs, mice, rats, hamsters, rabbits, guinea pigs, captive animals such as zoo animals, and wild animals. As used herein the term "tissue" refers to an organ or set of specialized cells such as skin tissue, lung tissue, kidney tissue, and other types of cells.

The term "therapeutically effective amount" means an amount of therapeutic agents, or a rate of delivery of such therapeutic agents, effective to facilitate a desired therapeutic effect. The precise desired therapeutic effect will vary according to the mucositis related condition to be treated, the formulation to be administered, and a variety of other factors that are appreciated by those of ordinary skill in the art.

### Methods of treatment

One aspect of the application provides methods for treating, preventing, or reducing the severity of mucositis in a patient, the method comprising administering to a patient in need thereof, a therapeutically effective amount of an SAP polypeptide.

In some embodiments, administration of an SAP polypeptide reduces the number of days a patient is afflicted with mucositis. In some embodiments, administration of an SAP agonist delays the development of mucositis. Mucositis that results as a side effect to cancer treatment, generally appears a few days after the start of treatment and can take two to four weeks after cessation of treatment to clear. In some embodiments, administration of an SAP polypeptide delays the development of mucositis by at least 1, 2, 3, 4, 5, 6, 7, 8, 9, 10, 15, or more days. In some embodiments, administration of an SAP polypeptide reduces the number of days a patient is afflicted with mucositis by at least 1, 2, 3, 4, 5, 6, 7, 8, 9, 10, 15, or more days. In some embodiments, administration of an SAP agonist speeds the resolution of mucositis.

In some embodiments, administration of an SAP agonist reduces the severity of mucositis by at least one grade according to the National Cancer Institute-Common Toxicity Criteria (NCI-CTC). Various assessment scales exist for the objective classification of mucositis, including the World Health Organization, the Radiation Therapy Oncology Group, the visual analog scale, the oral mucositis scale and the oral mucositis assessment scale, see Sonis, ST, et al. Cancer. 100:1995-2025 (2004) for review. The NCI-CTC provides a grading system from 1-5 for oral mucositis and from 1-4 for gastrointestinal mucositis. A person skilled in the art is capable of assessing the disease progression in a patient to determine the severity of mucositis.

An SAP polypeptide may be administered to subjects who have recently received or are likely to receive a dose of radiation or toxin. In one embodiment, the dose of radiation or toxin is received as part of a work-related or medical procedure, e.g., working in a nuclear power plant, flying an airplane, an X-ray, CAT scan, or the administration of a radioactive dye for medical imaging; in such an embodiment, the SAP polypeptide is administered as a prophylactic measure. In another embodiment, the radiation or toxin exposure is received unintentionally, e.g., as a result of an industrial accident, habitation in a location of natural radiation, terrorist act, or act of war involving radioactive or toxic material. In such a case, the SAP polypeptide is preferably administered as soon as possible after the exposure to inhibit apoptosis and the subsequent development of acute radiation syndrome.

While the methods can be used to treat patients afflicted with mucositis, in some embodiments, the methods are also carried out with patients who do not have, but are at risk of developing mucositis (e.g., cancer or other patients scheduled to receive, currently receiving, or previously treated with radiation and/or chemotherapy). In patients at risk of developing mucositis, treatment according to the invention can reduce the severity of mucositis resulting from their treatment, inhibit the development of mucositis, or prevent the onset of mucositis. In some embodiments, treatment with an SAP polypeptide delays the onset of symptoms of mucositis by at least 1, 2, 3, 4, 5, 6, 7, 8, 9, 10, 15, or more days. In some embodiments, treatment reduces the number of days a patient is afflicted with mucositis by at least 1, 2, 3,4, 5, 6, 7, 8, 9, 10, 15, or more days. In some embodiments, treatment reduces the severity of mucositis by at least one grade according to the NCI-CTC grading system.

According to the methods of the invention, an SAP polypeptide is administered to a patient before, during, and/or after treatment with a therapy that causes mucositis (e.g., oral, alimentary or gastrointestinal mucositis) or puts the patient at risk of developing such mucositis. As is noted above, such treatments include radiation and chemotherapy, which act by blocking the growth of rapidly dividing cells, such as cancer cells and epithelial cells that line the surfaces of the gastrointestinal, respiratory, and genitourinary tracts. Specific examples of treatments that can lead to mucositis include radiation treatment (e.g., head and/or neck, whole body, targeted, and/or hyperfractionated radiation), as well as chemotherapeutic regimens used in the treatment of, or as adjuvant treatments for, conditions such as breast cancer, colon cancer, gastric cancer, genitourinary (e.g., bladder, prostate, or testicular) cancer, gynecologic (e.g., cervical, endometrial, ovarian, or uterine) cancer, head and neck/esophageal cancer, leukemia, lung (small cell or non small-cell) cancer, lymphoma (Hodgkin's or non-Hodgkin's), melanoma, multiple myeloma, pancreatic cancer, and sarcoma. Myeloablative therapy in preparation for bone marrow transplantation can also lead to mucositis.

As is known in the art, cancers such as these can be treated using approaches involving immunotherapy by use of agents such as, for example, rituximab, cetuximab, or bevacizumab, alone or in combination with chemotherapy or radiation therapy. In other examples, chemotherapeutic approaches that may induce mucositis include those utilizing (either as single agents or in combinations) platinum derivatives such as carboplatin, cisplatin, and oxaplatin; mitosis inhibitors such as paclitaxel, docetaxel, vinorelbine, vincristine, and vinblastine; topoisomerase inhibitors such as etoposide, irinotecan, and topotecan; antimetabolites such as gemcitabine, capecitabine, fludarabine, methotrexate, 5-fluorouracil, cladribine, pentostatin, and cytarabine; DNA synthesis inhibitors such as doxorubicin, epirubicin, idarubicin, daunorubicin, bleomycin, mechlorethamine, and mitoxantrone; alkylating agents such as cyclophosphamide, ifosfamide, and melphalan carmustine; hormonal oncologics such as estramustine; and agents having other or unknown mechanisms such as dacarbazine. Use of these and other approaches to treating cancer is well known to those of skill in the art.

Treatment according to the invention can begin prior to cancer treatment (e.g., 1-2 days or 1 week prior to cancer treatment), at or near the same time as cancer treatment (e.g., simultaneously with, within 1-4 hours of, or on the same day as cancer treatment), or shortly after the cessation of cancer treatment (e.g., within 1-4 days after treatment cessation, and/or prior to or upon appearance of symptoms). Treatment can then be maintained, for example, until any symptoms of mucositis have substantially cleared or the risk of developing such symptoms has passed. Thus, treatment started before or at or near the same time as cancer treatment can be maintained, e.g., for several days. In other examples, treatment is maintained for 1, 2, 3, 4, or more weeks following the cessation of cancer treatment, as determined to be appropriate by one of skill in the art. In specific examples, the treatment according to the present invention is carried out prior to cancer treatment only; prior to and concurrently with cancer treatment only; prior to, concurrently with, and after cessation of cancer treatment; concurrently with cancer treatment only; concurrently with and after cessation of cancer treatment only; after cessation of cancer treatment only; or prior to and after cessation of cancer treatment only. Further, treatment according to the methods of the invention can be altered, stopped, or re-initiated in a patient, depending on the status of any symptoms of mucositis. Treatment can be carried out at intervals determined to be appropriate by those of skill in the art. For example, the administration can be carried out 1, 2, 3, or 4 times or more/day.

The application provides SAP polypeptides useful for the treatment of mucositis and mucositis related disorders. Mucositis may be oral, gastrointestinal, or esophageal. Disorders that may be treated with SAP agonists include gastric and duodenal ulcers, erosions of the stomach and esophagus, colitis, gastroesophageal reflux, and inflammatory bowel disease. In some embodiments, the inflammatory bowel disease is Crohn's disease or ulcerative colitis.

### (i) Human serum amyloid P

In certain embodiments, the SAP polypeptide is SAP comprising five human SAP protomers (SEQ ID NO: 1). The term "SAP protomer" is intended to refer to a polypeptide that is at least 60%, at least 70%, at least 80%, at least 85%, at least 90%, at least 95%, at least 97%, at least 99% or 100% identical to human SAP protomer (SEQ ID NO:1), as determined using the FASTDB computer program based on the algorithm of Brutlag et al. (Comp. App. Biosci., 6:237-245 (1990)). In a specific embodiment, parameters employed to calculate percent identity and similarity of an amino acid alignment comprise: Matrix=PAM 150, k-tuple=2, Mismatch Penalty=1, Joining Penalty=20, Randomization Group Length=0, Cutoff Score=1, Gap Penalty=5 and Gap Size Penalty=0.05. The term "SAP protomer" encompasses functional fragments and fusion proteins comprising any of the preceding. Generally, an SAP protomer will be designed to be soluble in aqueous solutions at biologically relevant temperatures, pH levels and osmolarity. The protomers that non-covalently associate together to form SAP may have identical amino acid sequences and/or post-translational modifications or, alternatively, individual protomers may have different sequences and/or modifications.

Some aspects of the invention provide polypeptides, or provide therapeutic methods for employing those polypeptides, wherein said polypeptides are defined, at least in part, to a reference sequence. Accordingly, such polypeptides may have a certain percentage of amino acid residues which are not identical to a reference sequence. In some embodiments, the non-identical residues have similar chemical properties to the residues to which they are not identical. Groups that have similar properties include the following amino acids: E, D, N, Q; H, K, R; Y, F and W; I, L, V, M, C, A; and S, T, C, P, A.

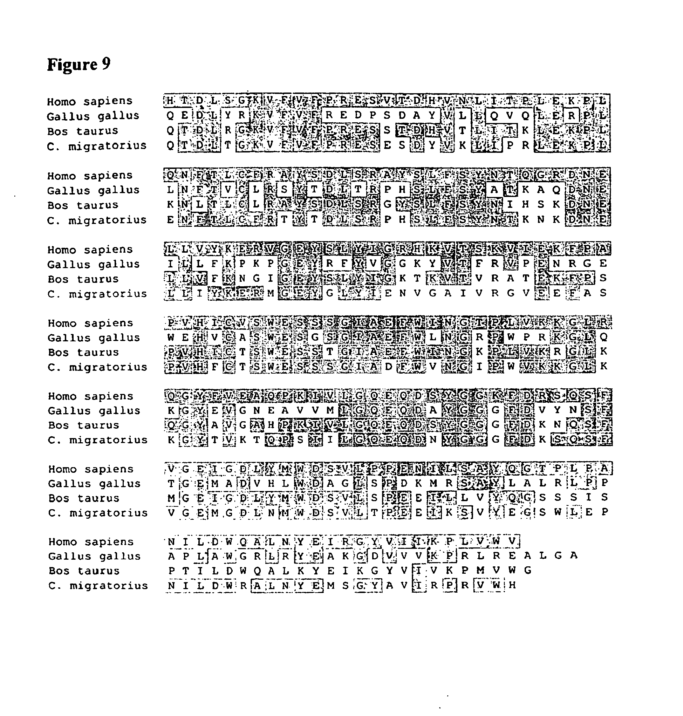

In some embodiments, the residues that are not identical are those which are not evolutionarily conserved between the reference sequence and an orthologous sequence in at least one evolutionarily related species, such as in species within the same order. In the case of a vertebrate reference sequence, the amino acids that may be mutated in a preferred embodiment are those that are not conserved between the reference sequence and the orthologous sequence in another vertebrate species. For example, if a polypeptide used in a method of the present invention is said to comprise an amino acid sequence that is at least 95% identical to human SAP (SEQ ID NO: 1), then said polypeptide may have non-identical residues to those positions in which the human SAP and that of another vertebrate differ. Figure 9 depicts human SAP aligned against two mammalian and one avian SAP sequence. Unshaded residues indicate residues that differ from the human SAP sequence.

Polypeptides sharing at least 95% identity with SEQ ID NO:1 include polypeptides having conservative substitutions in these areas of divergence. Typically seen as conservative substitutions are the replacements, one for another, among the aliphatic amino acids Ala, Val, Leu, and Ile, interchange of the hydroxyl residues Ser and Thr, exchange of the acidic residues Asp and Glu, substitution between the amide residues Asn and Gln, exchange of the basic residues Lys and Arg and replacements among the aromatic residues Phe, Tyr. Additional guidance concerning which amino acid changes are likely to be phenotypically silent can be found in Bowie et al., Science 247:1306-1310 (1990).

In certain embodiments, SAP polypeptides comprising polymers that are at least 60%, at least 70%, at least 80%, at least 85%, at least 90%, at least 95%, at least 97%, or at least 99% identical to SEQ ID NO. 1 increase SAP signaling.

In certain embodiments, an SAP signaling agonist is an SAP variant. The term "SAP variant" is intended to refer to a protein comprising from two to five SAP protomers that demonstrates one or more of the following features as compared to the human SAP pentamer: increased plasma half-life, increased in vitro stability, or increased in vivo stability relative to human SAP.

In specific embodiments of the present invention, compositions containing SAP, may be operable to raise SAP concentration in target locations to approximately at least 0.5 µg/ml. In humans, I¹²⁵ radiolabelled SAP has been previously administered to study patients with amyloidosis. In the treatments, approximately 600 µg of SAP was administered to an adult human. Accordingly, administration of approximately 600 µg of SAP systemically to an adult human is safe. Higher dosages may also be safe under appropriate conditions.

### Pharmaceutical Preparations and Formulations

In certain embodiments, the methods described herein involve administration of an SAP polypeptide to a subject. The SAP polypeptides may be formulated in a conventional manner using one or more physiologically acceptable carriers or excipients. For example, SAP polypeptides may be formulated for administration by, for example, injection (e.g. SubQ, IM, IP), inhalation or insufflation (either through the mouth or the nose) or oral, buccal, sublingual, transdermal, nasal, parenteral or rectal administration. In certain embodiments, SAP agonists may be administered locally, at the site where the target cells are present, i.e., in a specific tissue, organ, or fluid (e.g., blood, cerebrospinal fluid, tumor mass, etc.).

SAP polypeptides can be formulated for a variety of modes of administration, including systemic and topical or localized administration. Techniques and formulations generally may be found in Remington's Pharmaceutical Sciences, Meade Publishing Co., Easton, PA. For parenteral administration, injection is preferred, including intramuscular, intravenous, intraperitoneal, and subcutaneous. For injection, the compounds can be formulated in liquid solutions, preferably in physiologically compatible buffers such as Hank's solution or Ringer's solution. In addition, the compounds may be formulated in solid form and redissolved or suspended immediately prior to use. Lyophilized forms are also included.

For oral administration, the pharmaceutical compositions may take the form of, for example, tablets, lozanges, or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., ationd oil, oily esters, ethyl alcohol or fractionated vegetable oils); and preservatives (e.g., methyl or propyl-p-hydroxybenzoates or sorbic acid). The preparations may also contain buffer salts, flavoring, coloring and sweetening agents as appropriate. Preparations for oral administration may be suitably formulated to give controlled release of the active compound.

For administration by inhalation (e.g., pulmonary delivery), SAP agonists may be conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g., gelatin, for use in an inhaler or insufflator may be formulated containing a powder mix of the compound and a suitable powder base such as lactose or starch.

SAP polypeptides may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

In addition, SAP polypeptides may also be formulated as a depot preparation. Such long acting formulations may be administered by implantation (for example subcutaneously or intramuscularly) or by intramuscular injection. Thus, for example, SAP polypeptides may be formulated with suitable polymeric or hydrophobic materials (for example as an emulsion in an acceptable oil) or ion exchange resins, or as sparingly soluble derivatives, for example, as a sparingly soluble salt. Controlled release formula also includes patches.

In certain embodiments, SAP polypeptides are incorporated into a topical formulation containing a topical carrier that is generally suited to topical drug administration and comprising any such material known in the art. The topical carrier may be selected so as to provide the composition in the desired form, e.g., as an ointment, lotion, cream, microemulsion, gel, oil, solution, or the like, and may be comprised of a material of either naturally occurring or synthetic origin. It is preferable that the selected carrier not adversely affect the active agent or other components of the topical formulation. Examples of suitable topical carriers for use herein include water, alcohols and other nontoxic organic solvents, glycerin, mineral oil, silicone, petroleum jelly, lanolin, fatty acids, vegetable oils, parabens, waxes, and the like.

Pharmaceutical compositions may comprise from about 0.00001 to 100% such as from 0.001 to 10% or from 0.1% to 5% by weight of one or more SAP polypeptides described herein. In certain topical formulations, the active agent is present in an amount in the range of approximately 0.25 wt. % to 75 wt. % of the formulation, preferably in the range of approximately 0.25 wt. % to 30 wt. % of the formulation, more preferably in the range of approximately 0.5 wt. % to 15 wt. % of the formulation, and most preferably in the range of approximately 1.0 wt. % to 10 wt. % of the formulation.

An example of formulation for human SAP (hSAP) would be 0.5-15 mg/mL hSAP dissolved in water for injection containing 1-20 mM Tris, 140 mM NaCl with buffer to a pH of 7.0-9.0. A more preferred example of formulation for human SAP (hSAP) would be 5 mg/mL hSAP dissolved in water for injection containing 10 mM Tris, 140 mM NaCl with phosphate buffer to a pH of 8.0.

SAP polypeptides described herein may be stored in oxygen free environment according to methods in the art.

### Combination treatment

The methods of the invention can be used alone or in conjunction with other approaches for reducing the severity of mucositis. For example, the methods of the invention can be carried out in combination with antimicrobial or antifungal therapies, e.g., therapies involving administration of antibiotics such as nystatin, amphotericin, acyclovir, valacyclovir, clotimazole, fluconazole, and tetracycline compounds (include doxycycline, minocycline, tetracycline, oxytetracycline, chlortetracycline, demeclocycline, lymecycline, and sancycline). As a specific example of such treatment, patients with head and neck cancer receiving radiotherapy have colonization of the oropharyngeal region with gram-negative bacteria. Selective decontamination of the oral cavity with anti-microbial agents has the benefit of reducing oral mucositis associated with radiation therapy, but there may be limitations to the beneficial effects of such treatment. Anti-microbial therapy can kill bacteria, but cannot reduce endotoxin, and indeed may actually increase endotoxin. As endotoxin is a potent mediator of inflammation, it may contribute to the aggravation of mucositis and, thus, co-treatment with an antiendotoxin compound (e.g., a Lipid A analog, such as eritoran) and antibiotics can be used as a more effective approach to treating oral mucositis in such patients, according to the invention.

Antibiotic lozenges designed to dissolve in the mouth and decontaminate the oral mucosa have been developed and have been widely recommended to reduce oral infections associated with mucositis. The lozenges may contain polymixin E, tobramycin and amphotericin B, which together provide broad spectrum antibacterial and antifungal cover. These are commonly known as PTA lozenges or PTA pastilles. There is evidence supporting the use of PTA lozenges in preventing infectious complications of mucositis in cancer patients undergoing radiotherapy.

Some anti-viral therapies have been developed to treat patients suffering from mucositis that have an underlying viral infection. Acyclovir is an antiviral agent which is active against the Herpes species that commonly infect the oral mucous membranes in immunosuppressed cancer patients.

IL-11 has been investigated to mitigate the mucotoxic effects of radiation therapy and chemotherapy and have demonstrated activity in small animal trials. According to the present invention, IL-11, analogs, and derivatives thereof, are administered to patients conjointly with SAP agonists, either prophylactically or at the onset of symptoms associated with the aforementioned disorders. IL-11 and SAP polypeptides can be administered in suitable pharmaceutically acceptable carriers either alone or in combination with other conventional agents useful in alleviating the symptoms associated with the aforementioned disorders.

In one embodiment, the present invention comprises preparations of IL-11 and SAP polypeptides that are suitable for oral delivery to the mouth. Suitable oral preparations may be prepared with aqueous-based solutions such as sodium bicarbonate (e.g., Brioschi.RTM.), or in gels and suspensions for topical administration in the mouth. Oral preparations may also take the form of patches for delivery of IL-11 and SAP polypeptides agonists to the mouth via sustained release. Additional oral preparations may comprise IL-11 and SAP polypeptides in the form of a lozenge or an uncoated tablet which is retained in the mouth. The oral preparations are particularly well-suited for disorders and inflammatory responses involving the mucosa of the head, neck and/or mouth. Such conditions include oral mucositis. Such conditions may result, for example, from chemotherapy or radiotherapy for head and neck cancer, cervical esophageal cancer or lung cancer.

In other embodiments, the present invention comprises preparations of IL-11 and SAP polypeptides which are suitable for topical delivery for mucosa and/or dermis. Such topical preparations may be prepared in the form of aqueous-based solutions, gels, ointments or creams for topical administration, as gels and suspensions for cervical administration, as pills, tablets, capsules or suppositories for immediate or sustained release to the gastrointestinal tract, or in the form of solution for enema. Such topical preparations are especially suited for treatment of disorders relating to local regions, such gastrointestinal mucositis. Some of these conditions may result, for example, from chemotherapy and/or radiotherapy for colorectal cancer, prostate cancer, cervical esophageal cancer or lung cancer. Among the reasons that local administration may be preferred are the ease of administering a topical formulation compared to administration of subcutaneous injectable formulations. In certain classes of patients, the toxicity profile of chemotherapeutic agents may be such that concurrent parenteral administration of IL-11 is relatively unsuited. Other patients may have medical conditions for which the adverse event profile of parenteral IL-11 is relatively unsuited.

Suitable doses of IL-11 agonists may be administered from once a week up to about six times daily. Treatment may continue for a period of between one day and six months, or for as long as is deemed necessary and safe in the treatment of the aforementioned disorders, as is readily ascertained by standard tests by the attending physician, depending upon the nature of the disorder being treated.

The methods of the invention can also be used in conjunction with palliative therapies including the use of topical rinses, gels, or ointments that include lidocaine, articaine, and/or morphine, as well as other analgesic or anti-inflammatory agents. Specific examples of other agents and approaches that can be used in combination with SAP agonists, according to the methods of the invention, include the following: velafermin (CG53135/fibroblast growth factor-20/FGF-20, CuraGen), palifermin (recombinant keratinocyte growth factor; rHuKGF; Kepivance™; Amgen) and AES-14 (uptake-enhanced L-glutamine suspension)(Peterson, J. Support Oncol. 4(2 Suppl. 1)9-13, 2006); oral cryotherapy (including beta-carotene, vitamin E, oxpentifylline, azelastine hydrochloride, and prostaglandins E1 and E2), low-level laser therapy, chlorhexidine, amifostine, hematologic growth factors, pentoxifylline, and glutamine (Saadeh, Pharmacotherapy 25(4):540-554, 2005); amifostine, antibiotic paste or pastille, hydrolytic enzymes, ice chips, benzydamine, calcium phosphate, honey, oral care protocols, povidone, and zinc sulphate (Worthington et al., Cochrane Database Syst. Rev. 2:CD000978, 2006); flurbiprofen (e.g., administered as a tooth patch; Stokman et al., Support Care Cancer 13(1):42-48, 2005); diphenhydramine, magnesium hydroxide/aluminum hydroxide, nystatin, and corticosteroids (Chan et al., J. Oncol. Pharm. Pract. 11 (4):139-143, 2005) including prednisone (for short term systemic therapy), flucinonide and globetasol (for topical therapy), and elixir dexamthasone (for oral therapy); oral transmucosal fentanyl citrate (e.g., administered in the form of a lozenge; Shaiova et al., Support Care Cancer 12(4):268-273, 2004); clonazepam (e.g., in the form of a tablet; Gremeau-Richard et al., Pain 108(102):51-57, 2004); capsaicin (e.g., in the form of a lozenge; Okuno et al., J. Cancer Integr. Med. 2(3): 179-183, 2004); ketamine (e.g., in the form of an oral rinse; Slatkin et al., Pain Med. 4(3):298-303, 2003); and granulocyte-macrophage colony-stimulating factor (GM-CSF)/granulocyte colony-stimulating factor (G-CSF), Transforming Growth Factor-B 3 (TGF-B 3), keratinocyte growth factor 1, laser light therapy, N-acetylcyteine (NAC, Lappas, 2003. J. Clin Endocrinoloyg Metab) and glutamine supplements (Duncan et al., Aliment. Pharmacol. Ther. 18(9):853-874, 2003); mucosal barrier and coating agents (including sueralfate, sodium alginante, kaolin-pectin, plastic wrap film, radiation guards, and antacids); chamomile; allouprinol; propantheline; silver nitrate COX-1 or COX-2 antagonist (including indomethacin and flurbriprofin); IL-6 antagonist, a TNF-α antagonist; L-1 antagonist, interferon-gamma antagonist; NO antagonist (aminoguanidine and guanidine); mast cell antagonist (antihistamines, serine protease inhibitors, or degranulation inhibitors); aminophosphorothioate or aminoalkyl thiol compounds; resveratrol; NF-κB antagonist; angiotensinogen, angiotensin I (AI), AI analogues, AI fragments and analogues thereof, angiotensin II (AII), AII analogues, AII fragments or analogues thereof, or AII AT.sub.2 type 2 receptor agonists; bismuth-containing compound (bismuth salt or bismuth complex)

In certain embodiments, the methods described herein involve administration of an anti-mucositis therapeutic and a SAP agonist. These therapeutic agents may be formulated to be administered conjointly using one or more physiologically acceptable carriers. These combinational therapeutics can be formulated for a variety of modes of administration, including systemic and topical or localized administration as described herein for the administration of SAP and SAP agonists.

In certain embodiments, the anti-mucositis therapeutic may be formulated for sustained release of the SAP and/or and additional anti-mucositis therapeutic. Formulations for sustained release may be administered using one or more physiologically acceptable carriers. These combinational therapeutics can be formulated for a variety of modes of administration, including systemic and topical or localized administration as described herein for the administration of SAP and SAP polypeptide. In certain embodiments, the anti-mucositis therapeutic may be administer to a patient to prevent or reduce the severity of oral mucositis.

The present invention provides anti-mucositis therapeutic comprising an orally acceptable carrier. As used herein, an "orally acceptable carrier" refers to a material or combination of materials that are safe for use in the compositions of the present invention, commensurate with a reasonable benefit/risk ratio, with which the anti-mucositis therapeutic may be associated while retaining significant efficacy. Preferably, the carrier does not substantially reduce the efficacy of the anti-mucositis therapeutic. Selection of specific carrier components is dependant on the desired product form, including dentifrices, rinses, gels, and paints. In various embodiments, the carrier is operable to sufficiently adhere the anti-mucositis therapeutic against surfaces within the oral cavity to which the composition is administered, without concomitant use of a dental tray, mouthpiece, tape, or similar appliance. In various embodiments, the carrier is operable for use with a tape, tray, mouthpiece or similar appliance. In some embodiments, the anti-mucositis therapeutic includes SAP polypeptides in an orally acceptable formulation. In some embodiments, the anti-mucositis therapeutic includes SAP polypeptide and additional active agents in an orally acceptable formulation.

Materials among those that are useful in carriers include adhesion agents, viscosity modifiers, diluents, surfactants, foam modulators, peroxide activators, peroxide stability agents, abrasives, pH modifying agents, humectants, mouth feel agents, sweeteners, flavorants, colorants, and combinations thereof. It is understood that while general attributes of each of the above categories of materials may differ, there may be some common attributes and any given material may serve multiple purposes within two or more of such categories of materials. Preferably, such carrier materials are selected for compatibility with the anti-mucositis therapeutic agent and with other ingredients of the composition.

In some embodiments, concurrent administration of an SAP polypeptide and an additional agent, such as velafermin, palifermin, or an antimicrobial or antifungal agent, reduces the effective therapeutic dosage of the SAP polypeptide and/or the additional agent as compared to the dosage required for a therapeutic effect when administered alone. In some embodiments, the concurrent administration further reduces the number of days a patient is afflicted with mucositis as compared to administration with an SAP polypeptide or the additional agent alone. In some embodiments, the concurrent administration further delays the development of mucositis in a patient as compared to administration with an SAP polypeptide or the additional agent alone. In some embodiments, the concurrent administration further reduces the severity of mucositis in a patient as compared to administration with an SAP polypeptide or the additional agent alone.

### SEQUENCE LISTING

SEQ ID NO: 1 human serum amyloid protein P
HTDLSGKVFVFPRESVTDHVNLITPLEKPLQNFTLCFRAYSDLSRAYSLFSYNTQGRDN
ELLVYKERVGEYSLYIGRHKVTSKVIEKFPAPVHICVSWESSSGIAEFWINGTPLVKKGL
RQGYFVEAQPKIVLGQEQDSYGGKFDRSQSFVGEIGDLYMWDSVLPPENILSAYQGTP
LPANILDWQALNYEIRGYVIIKPLVWV

SEQ ID NO: 2 Gallus gallus serum amyloid protein P
QEDLYRKVFVFREDPSDAYVLLQVQLERPLLNFTVCLRSYTDLTRPHSLFSYATKAQD
NEILLFKPKPGEYRFYVGGKYVTFRVPENRGEWEHVCASWESGSGIAEFWLNGRPWPR
KGLQKGYEVGNEAVVMLGQEQDAYGGGFDVYNSFTGEMADVHLWDAGLSPDKMRS
AYLALRLPPAPLAWGRLRYEAKGDVVVKPRLREALGA

SEQ ID NO: 3 Bos taurus serum amyloid protein P
QTDLRGKVFVFPRESSTDHVTLITKLEKPLKNLTLCLRAYSDLSRGYSLFSYNIHSKDNE
LLVFKNGIGEYSLYIGKTKVTVRATEKFPSPVHICTSWESSTGIAEFWINGKPLVKRGLK
QGYAVGAHPKIVLGQEQDSYGGGFDKNQSFMGEIGDLYMWDSVLSPEEILLVYQGSSS
ISPTILDWQALKYEIKGYVIVKPMVWG

SEQ ID NO: 4 Cricetulus migratorius serum amyloid protein P
QTDLTGKVFVFPRESESDYVKLIPRLEKPLENFTLCFRTYTDLSRPHSLFSYNTKNKDNE
LLIYKERMGEYGLYIENVGAIVRGVEEFASPVHFCTSWESSSGIADFWVNGIPWVKKGL
KKGYTVKTQPSIILGQEQDNYGGGFDKSQSFVGEMGDLNMWDSVLTPEEIKSVYEGS
WLEPNILDWRALNYEMSGYAVIRPRVWH

### EXEMPLIFICATION

### Example 1.

Human SAP (hSAP) was evaluated in a unique rat model of radiation-induced enteropathy (Hauer-Jensen, 1988). Briefly, a bilateral orchiectomy was performed on male rats and a loop of the small intestine was sutured to the inside of the scrotum, producing an artificial hernia. On study day 0, a single 17 Gy dose of localized irradiation was delivered to the short segment of small intestine located inside the scrotum, while the remainder of the intestine was shielded from damage. Rats were dosed i.p. with 480 µg of hSAP (n=15) or vehicle (n=17) immediately following irradiation; subsequent doses were administered on Days 2, 4, 6, 8, 10 and 12. Rats were sacrificed on day 14, and sections of irradiated and shielded intestine were processed for histologic evaluation. A histopathologic radiation injury score (RIS) (Hauer-Jensen, 1983) comprising 7 parameters of early (2 weeks) and late developing (12 wk-26 wk) enteropathy was used to quantify radiation injury. Quantitative immunohistochemistry, total collagen content and RT-PCR were also conducted.

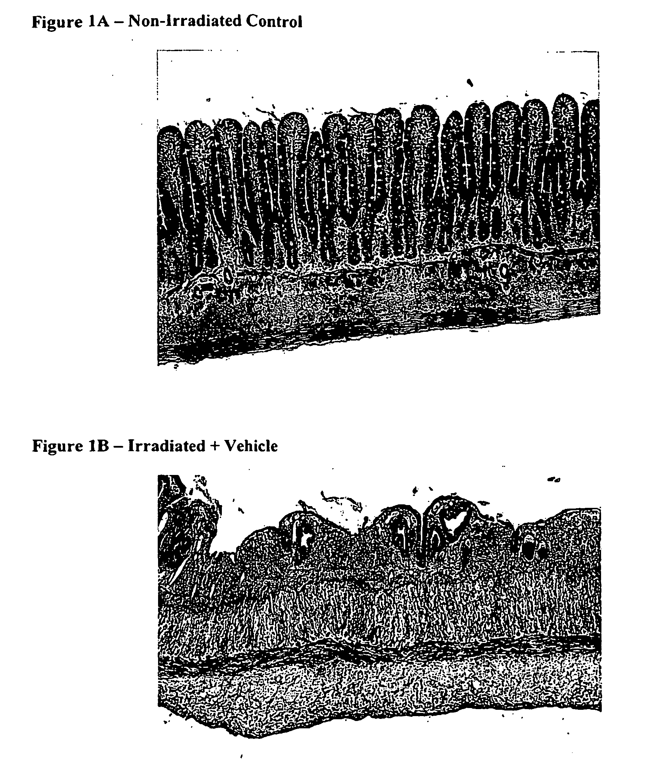

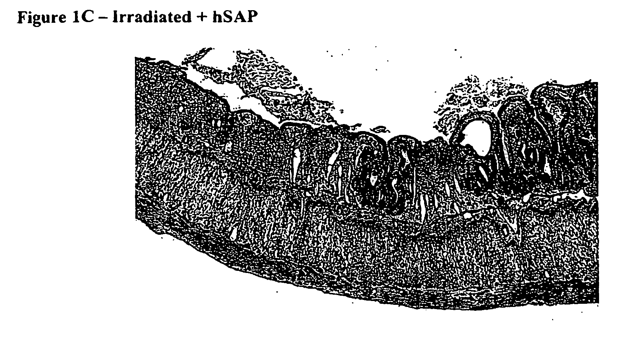

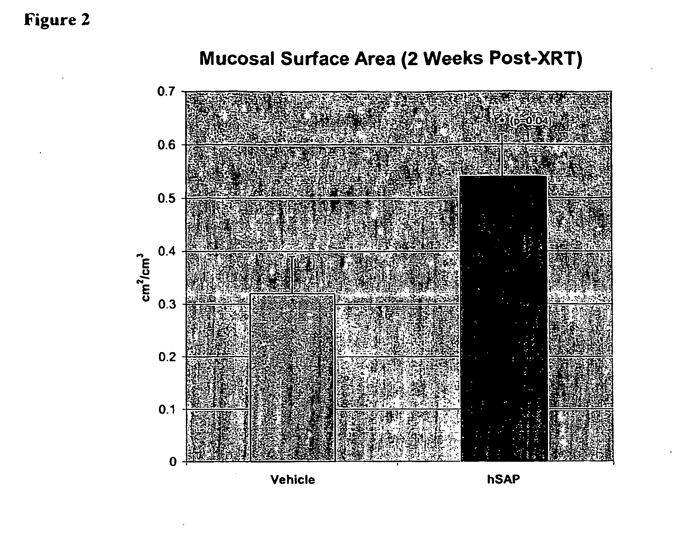

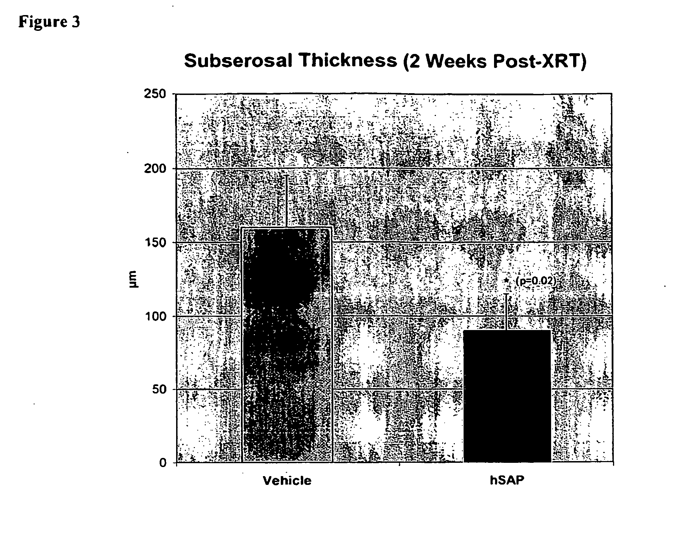

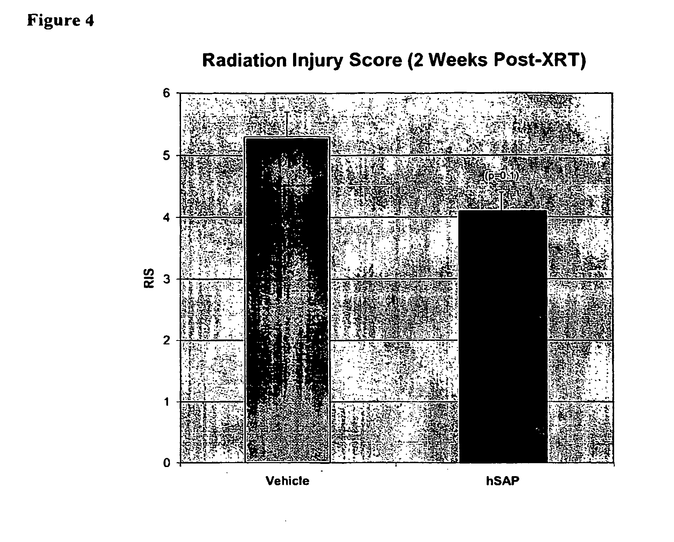

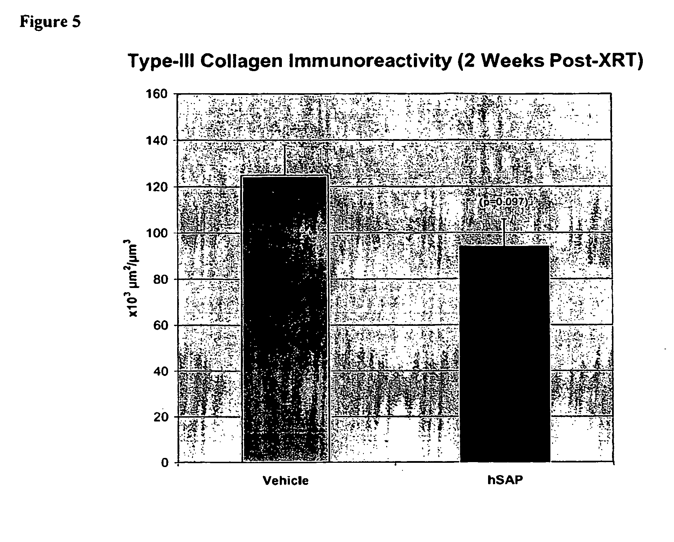

Hematoxylin and eosin (H&E) staining of intestines from non-irradiated control, irradiatated vehicle-treated and hSAP-treated rats are shown in Figure 1. Epithelial atypia with abnormally oriented crypt cells, mucosal inflammation with areas of severe ulceration, reduced mucosal surface area, and significant subserosal thickening were observed in vehicle-treated animals. While mucosal damage and inflammation were evident in hSAP-treated animals, total mucosal surface area was significantly preserved (Figure 2) and subserosal thickening was significantly reduced (Figure 3). RIS was reduced in hSAP-treated animals (Figure 4), but did not reach significance (p=0.1). Similarly, reduction of type-III collagen immunoreactivity (Figure 5) did not reach significance (p=0.097).

Several parameters (vascular sclerosis, fibrosis including collagen deposition, lymphatic congestion and ileitis profunda) were used to calculate the radiation injury score developed during the intermediate (8 wk) and late periods (26 wk) of radiation enteropathy. The 14-day recovery period utilized in this current study did not allow for observation of these late-developing injuries. Extending recovery time beyond the acute injury phase is expected to increase the impact of SAP on both RIS and type-III collagen.

Hauer-Jensen, M., L. Poulakos, et al. (1988). "Effects of accelerated fractionation on radiation injury of the small intestine: a new rat model." Int J Radiat Oncol Biol Phys 14(6): 1205-12.

Hauer-Jensen, M., T. Sauer, et al. (1983). "Late changes following single dose roentgen irradiation of rat small intestine." Acta Radiol Oncol 22(4): 299-303.

### Example 2.

Human SAP (hSAP) was evaluated in a hamster cheek pouch model of radiation-induced oral mucositis (Sonis, 1990). Briefly, five- to six-week-old male Golden Syrian hamsters weighing 80-90 grams were exposed to a single 40 Gy dose of localized radiation on Day 0. Animals were anesthetized with IP ketamine/xylazine, the left buccal pouch was everted, and the rest of the body was protected with a lead shield. Radiation was generated with a 160 kilovolt potential source at a focal distance of 21 cm, hardened with a 3.0 mm Al filtration system, and delivered at a rate of 1.32 Gy/minute.

Hamsters (n=16/group) were dosed IP with vehicle (Group 1) or 2 mg/kg hSAP (Group 2) every other day beginning 30 minutes prior to radiation treatment for a total of 7 doses (final dose on Day 12). Eight additional animals (Group 3) served as age-matched controls (no radiation, no treatment).

Mucositis was evaluated clinically beginning on Day 6, continuing on alternate days until Day 28, then weekly until terminal sacrifice on Day 45. Animals were lightly anesthetized with inhaled anesthetic, and the left pouch was everted and photographed. At the end of the study, two blinded observers scored the photographs using a validated photographic scale (Sonis, 2000). A score ≥3 coincides with a clinically significant National Cancer Institute or World Health Organization score ≥3.

On Days 8 and 45, four hamsters from each study group were sacrificed and both buccal pouches were excised. On Days 16 and 28, four hamsters from groups 1 and 2 were sacrificed and both buccal pouches were excised. Pouches were fixed in 10% neutral buffered formalin and processed for histological evaluation. Slides were stained with H&E to examine fibrosis in the extracellular space. Slides were stained with Sirius red and analyzed with image analysis software to quantify total collagen deposition.

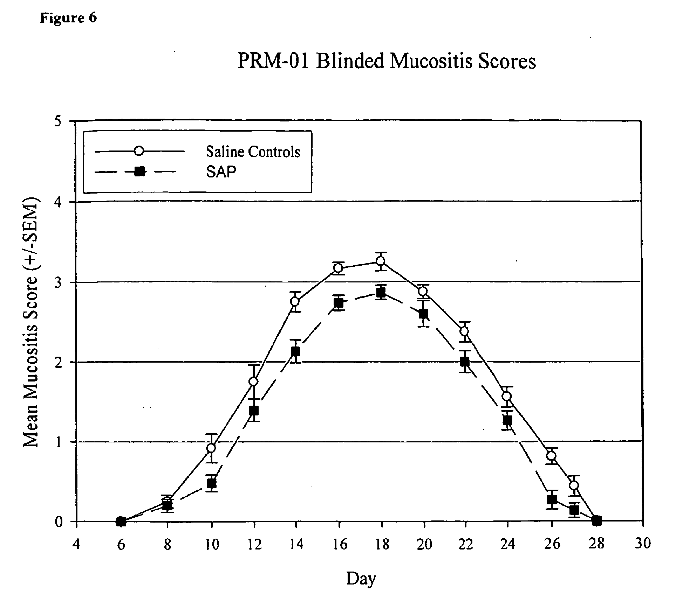

The mean blinded mucositis scores are presented in Figure 6. Mucositis peaked in the vehicle-treated animals on Day 18, with a mean score of 3.3. Both the day of peak effect and the mean peak score in vehicle-treated animals were consistent with historical data in this model. hSAP delayed the onset of mucositis and significantly reduced the mean peak score (2.9). The duration of mucositis, defined as the percentage of animal days spent with a score of ≥3, was significantly reduced in the hSAP-treated animals.

Sonis ST, Tracey C, Shklar G, Jenson J, Florine D. An animal model for mucositis induced by cancer chemotherapy. Oral Surgery. Oral Medicine, Oral Pathology. 69(4):437-43, 1990.

Sonis ST. Animal models of oral mucositis induced by antineoplastic drugs and radiation. In: Teicher B, ed. Tumor models in cancer research. Totowa, NJ: Humana Press; 2000.

### Example 3.

Human SAP (hSAP) was evaluated in a hamster cheek pouch model of radiation-induced oral mucositis to examine the effects of dose schedule and dose level on the frequency, severity, and duration of oral mucositis. Following the same procedure outlined in Example 2, five- to six-week-old male Golden Syrian hamsters were irradiated on Day 0 with 40 Gy of localized radiation to the left cheek pouch. Hamsters (n=12/group) were dosed ip with vehicle or hSAP according to the following table 1:

**Table 1**

| Group Number | Treatment | Schedule |
|---|---|---|
| 1 | PBS, ip | Days 0, 2, 4, 6, 8, 10 & 12 |
| 2 | hSAP, ip, 2 mg/kg | Days -1, 0, 2, 4, 6, 8, 10 & 12, 16, 18, 20, 22, 24 &26 |
| 3 | hSAP, ip, 2 mg/kg | Days 0, 1, 2, 3, 4, 5, 6, & 7 |
| 4 | hSAP, ip, 2 mg/kg | Days 0, 2, 4, 6, 8, 10 & 12 |
| 5 | hSAP, ip, 10 mg/kg | Days 0, 2, 4, 6, 8, 10 & 12 |

The Day 0 dose was administered 30 minutes prior to radiation. Mucositis was scored every other day beginning on Day 6, and continuing through Day 28, by anesthetizing the animals with an inhalation anesthetic and everting the cheek pouch. Mucositis was scored visually by comparison to a validated photographic scale, ranging from 0 for normal, to 5 for severe ulceration.

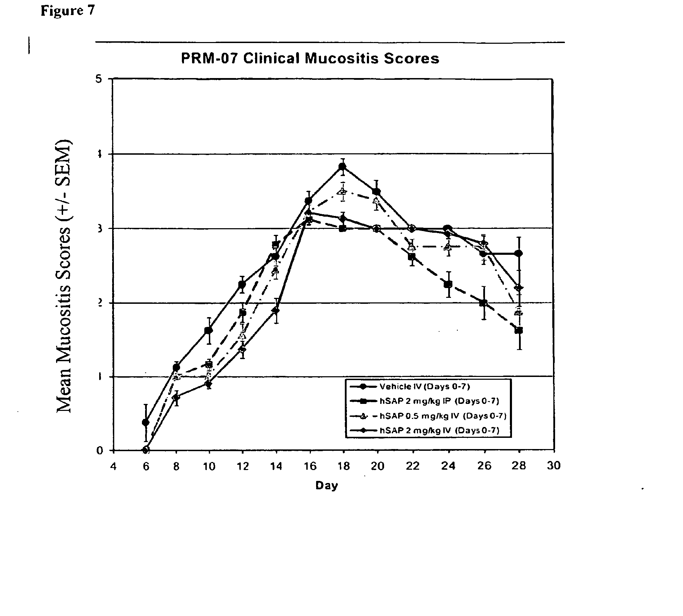

The mean blinded mucositis scores are presented in figure 7. Mucositis peaked in the vehicle treated group on Day 14, with a mean score of 3.5. Groups treated with hSAP had a delayed and lowered peak of mucositis. Treatment effects on mucositis were also evaluated by calculating the number of days with a mucositis score of 3 or higher. Chi-squared analysis of these data showed that the percentage of days with a score of 3 or higher was significantly reduced in all groups treated with hSAP (Table 2).

**Table 2.**

| Group Number | % Days with Mucositis Score ≥3 | P Value |
|---|---|---|
| 1 | 49.2 | - |
| 2 | 36.7 | 0.007 |
| 3 | 36.7 | 0.007 |
| 4 | 39.2 | 0.035 |
| 5 | 34.9 | 0.003 |

A further analysis of the mucositis scores was performed using the Mann-Whitney rank sum analysis to compare each hSAP treatment group to the vehicle control group. In this analysis, two days of significant reduction in the mucositis score are generally required before it is regarded as meaningful. Mucositis scores were significantly reduced on at least 4 days in all hSAP treatment groups relative to the vehicle control group (Table 3, significant differences identified in bold).

**Table 3.**

| Group Number | Study Day | | | | | | | | | | | |
|---|---|---|---|---|---|---|---|---|---|---|---|---|
| | 6 | 8 | 10 | 12 | 14 | 16 | 18 | 20 | 22 | 24 | 26 | 28 |
| 2 | 0.217 | 0.018 | **0.039** | 0.252 | 0.056 | 0.101 | 0.297 | 0.297 | 0.232 | 0.299 | **0.030** | **0.029** |
| 3 | 0.626 | 0.374 | 0.934 | 0.403 | **0.026** | 0.269 | 0.135 | 0.280 | 0.061 | **0.002** | **<0.001** | <0.001 |
| 4 | 0.217 | **0.021** | **0.006** | 0.287 | **0.003** | **0.048** | 0.230 | 0.116 | 0.072 | 0.461 | 0.030 | **0.003** |
| 5 | 0.138 | **0.010** | **0.061** | 0.285 | **<0.001** | 0.149 | 0.138 | 0.250 | 0.137 | 0.518 | **0.021** | **0.006** |

In conclusion, all hSAP treatment groups had significant reductions in the number of days with a mucositis score of 3 or higher when compared to vehicle controls. Group 5 (10 mg/kg hSAP on Days 0-12, q2d) had the fewest number of days with a mucositis score of three or more (76 days compared to 118 days in the vehicle treated group, p=0.003). Group 4 (2 mg/kg hSAP on Days 0-12, q2d) exhibited the greatest overall reduction in daily mucositis scores as determined using the Mann-Whitney rank sum test.

### Example 4

The effects of intravenous and intraperitoneal administration of hSAP were evaluated in a third study of radiation-induced oral mucositis. Hamsters in the intravenous dose groups were purchased with surgically implanted jugular catheters. Following the same procedure outlined in Example 2, five- to six-week-old male Golden Syrian hamsters were irradiated on Day 0 with 40 Gy of localized radiation to the left cheek pouch. Hamsters (n=12/group) were dosed with vehicle or hSAP according to the following table 4:

**Table 4.**

| Group Number | Treatment | Schedule |
|---|---|---|
| 1 | PBS, iv | Days 0, 1,2, 3, 4, 5, 6, & 7 |
| 2 | hSAP, ip, 2 mg/kg | Days 0, 1, 2, 3, 4, 5, 6, & 7 |
| 3 | hSAP, iv, 0.5 mg/kg | Days 0, 1, 2, 3, 4, 5, 6, & 7 |
| 4 | hSAP, ip, 2 mg/kg | Days 0, 1, 2, 3, 4, 5, 6, & 7 |

The Day 0 dose was administered 30 minutes prior to radiation. Mucositis was scored every other day beginning on Day 6, and continuing through Day 28, by anesthetizing the animals with an inhalation anesthetic and everting the cheek pouch. Mucositis was scored visually by comparison to a validated photographic scale, ranging from 0 for normal, to 5 for severe ulceration.

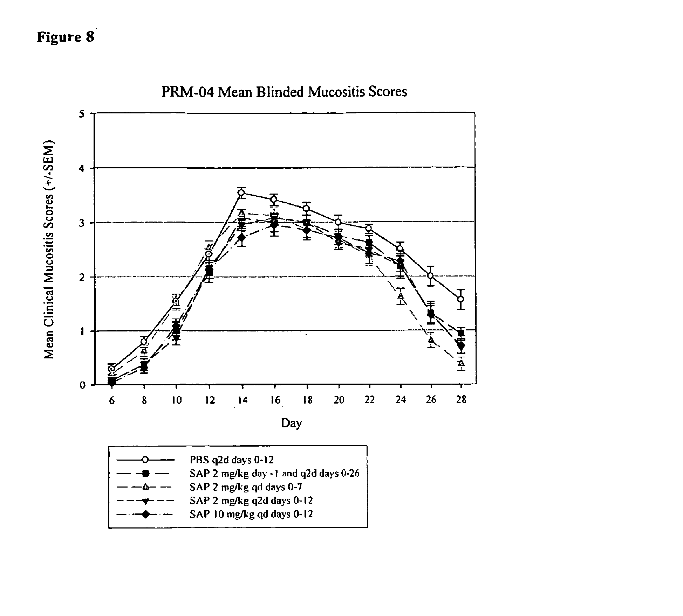

The mean blinded mucositis scores are presented in figure 8. Mucositis peaked in the vehicle treated group on Day 18, with a mean score of 3.8. The peak mucositis score was reduced in all hSAP treatment groups, although the day of peak effect was unchanged. Treatment effects on mucositis were also evaluated by calculating the number of days with a mucositis score of 3 or higher. Chi-squared analysis of these data showed that the percentage of days with a score of 3 or higher was significantly reduced in animals treated with ip hSAP, but was not significantly reduced in animals treated with iv hSAP (Table 5).

**Table 5.**

| Group Number | % Days with Mucositis Score ≥3 | P Value |
|---|---|---|
| 1 | 57.1 | - |
| 2 | 43.3 | 0.008 |
| 3 | 50.0 | 0.204 |
| 4 | 46.8 | 0.056 |

A further analysis of the mucositis scores was performed using the Mann-Whitney rank sum analysis to compare each hSAP treatment group to the vehicle control group. In this analysis, two days of significant reduction in the mucositis score are generally required before it is regarded as meaningful. Mucositis score was significantly reduced in all groups treated with hSAP relative to the vehicle control group (Table 6, significant differences identified in bold).

**Table 6**

| Group Number | Study Day | | | | | | | | | | | |
|---|---|---|---|---|---|---|---|---|---|---|---|---|
| | 6 | 8 | 10 | 12 | 14 | 16 | 18 | 20 | 22 | 24 | 26 | 28 |
| 2 | 0.512 | 0.512 | 0.061 | 0.154 | 0.353 | 0.273 | **<0.001** | **0.026** | 0.096 | **0.006** | 0.066 | **0.017** |
| 3 | 0.541 | 0.541 | **0.023** | **0.006** | 0.561 | 0.456 | **0.141** | **0.592** | 0.720 | 0.270 | 0.468 | 0.066 |
| 4 | 0.520 | 0.059 | **0.005** | **<0.001** | **0.006** | **0.492** | **0.002** | **0.026** | 0.981 | 0.588 | 0.726 | 0.236 |

In conclusion, hamster treated with 2 mg/kg hSAP, ip had significant reductions in the number of days with a mucositis score of 3 or higher when compared to vehicle controls (p=0.008). This group showed statistically significant improvements relative to vehicle-treated controls on Days 18, 20, 24 and 28. Hamsters with implanted catheters (Groups 1, 3 & 4) had signs of infection at the catheter sites, which may have influenced the resolution of mucositis. Hamsters treated with iv hSAP did not show an improvement in the number of days with a mucositis score of 3 or higher, but the groups did show significant improvements relative to vehicle-treated controls on several study days.

## Claims

1. A serum amyloid P (SAP) polypeptide for use in treating, preventing, or reducing the severity of mucositis or a mucositis-related disorder in a patient.

2. Use of an SAP polypeptide in the preparation of a medicament for treating, preventing, or reducing the severity of mucositis or a mucositis-related disorder in a patient.

3. The SAP polypeptide for use according to claim 1, or the use of claim 2, wherein the mucositis or mucositis related disorder is selected from gastrointestinal mucositis, oral mucositis, alimentary mucositis, esophageal mucositis, gastric ulcers, duodenal ulcers, erosions of the stomach and esophagus, colitis, gastroesophageal reflux, and inflammatory bowel disease.

4. The SAP polypeptide for use according to claim 1 or 3, or the use of one of claim 2 or 3, wherein the mucositis is associated with radiation treatment, chemotherapy, or a combination thereof.

5. The SAP polypeptide for use according to any one of claims 1, 3 or 4, or the use of any one of claims 2-4, wherein the patient is at risk of developing mucositis.

6. The SAP polypeptide for use according to claim 5, or the use of claim 5, wherein the SAP polypeptide is for administration prior to, concurrently with, or after a treatment that places the patient at risk of developing mucositis.

7. The SAP polypeptide for use according to claim 6, or the use of claim 6, wherein the treatment that places the patient at risk of developing mucositis comprises radiation therapy, chemotherapy, or a combination thereof.

8. The SAP polypeptide for use according to claim 6 or 7, or the use of of claim 6 or 7, wherein the SAP polypeptide is for treating mucositis or a mucositis-related disorder in a patient being treated for cancer.

9. The SAP polypeptide for use according to claim 8, or the use of claim 8, wherein the cancer is selected from head, neck, breast, lung, ovarian, prostate, lymphatic, leukemic, or gastrointestinal cancer.

10. The SAP polypeptide for use according to any one of claims 1 or 3-9, or the use of any one of claims 2-9, wherein the SAP polypeptide comprises an amino acid sequence that is at least 95% identical to SEQ ID NO: 1.

11. The use of claim 2, wherein the medicament further comprises a growth factor selected from velafermin or palifermin.

12. The SAP polypeptide for use according to any one of claims 1 or 3-9, or the use of any one of claims 2-9, wherein the SAP polypeptide comprises an amino acid sequence that is at least 97% identical to SEQ ID NO: 1.

13. The SAP polypeptide for use according to any one of claims 1 or 3-9, or the use of any one of claims 2-9, wherein the SAP polypeptide comprises an amino acid sequence that is at least 99% identical to SEQ ID NO: 1.

14. The SAP polypeptide for use according to any one of claims 1 or 3-9, or the use of any one of claims 2-9, wherein the SAP polypeptide comprises five polypeptides of SEQ ID NO: 1.

## Patentansprüche

1. Serum-Amyloid-P (SAP) Polypeptid zur Verwendung bei der Behandlung, der Prävention oder der Reduzierung des Schweregrads einer Mukositis oder einer der Mukositis verwandten Krankheit in einem Patienten.

2. Verwendung eines SAP-Polypeptids zur Herstellung eines Medikaments für die Behandlung, die Prävention oder die Reduzierung des Schweregrads einer Mukositis oder einer der Mukositis verwandten Krankheit in einem Patienten.

3. SAP-Polypeptid zur Verwendung nach Anspruch 1 oder Verwendung nach Anspruch 2, wobei die Mukositis oder die der Mukositis verwandte Krankheit gastrointestinale Mukositis, orale Mukositis, alimentäre Mukositis, Mukositis der Speiseröhre, Magengeschwür, Zwölffingerdarmgeschwür, Erosionen des Magens und der Speiseröhre, Colitis, gastroösophagealer Reflux oder chronischentzündliche Darmerkrankung ist/sind.

4. SAP-Polypeptid zur Verwendung nach Anspruch 1 oder 3 oder Verwendung nach Anspruch 2 oder 3, wobei die Mukositis mit einer Strahlenbehandlung, Chemotherapie oder einer Kombination davon assoziiert ist.

5. SAP-Polypeptid zur Verwendung nach einem der Ansprüche 1, 3 oder 4 oder Verwendung nach einem der Ansprüche 2-4, wobei der Patient gefährdet ist, eine Mukositis zu bekommen.

6. SAP-Polypeptid zur Verwendung nach Anspruch 5 oder Verwendung nach Anspruch 5, wobei das SAP-Polypeptid für die Verabreichung vor, zeitgleich mit oder nach einer Behandlung ist, die den Patienten gefährdet, eine Mukositis zu bekommen.

7. SAP-Polypeptid zur Verwendung nach Anspruch 6 oder Verwendung nach Anspruch 6, wobei die Behandlung, die den Patienten gefährdet, eine Mukositis zu bekommen eine Strahlenbehandlung, Chemotherapie oder eine Kombination davon umfasst.

8. SAP-Polypeptid zur Verwendung nach Anspruch 6 oder 7 oder Verwendung nach Anspruch 6 oder 7, wobei das SAP-Polypeptid zur Behandlung einer Mukositis oder einer der Mukositis verwandten Krankheit in einem Patienten, der gegen Krebs behandelt wird, ist.

9. SAP-Polypeptid zur Verwendung nach Anspruch 8 oder Verwendung nach Anspruch 8, wobei der Krebs Kopf-, Nacken-, Brust-, Lungen-, Gebärmutter-, Prostata-, Lymphgefäß-, Blut- oder Magen-Darm-Krebs ist.

10. SAP-Polypeptid zur Verwendung nach einem der Ansprüche 1 oder 3-9 oder Verwendung nach einem der Ansprüche 2-9, wobei das SAP-Polypeptid eine Aminosäuresequenz umfasst, die mindestens zu 95% identisch zur SEQ ID NO: 1 ist.

11. Verwendung nach Anspruch 2, wobei das Medikament weiterhin einen Wachstumsfaktor ausgewählt aus Velafermin oder Palifermin umfasst.

12. SAP-Polypeptid zur Verwendung nach einem der Ansprüche 1 oder 3-9 oder Verwendung nach einem der Ansprüche 2-9, wobei das SAP-Polypeptid eine Aminosäuresequenz umfasst, die mindestens zu 97% identisch zur SEQ ID NO: 1 ist.

13. SAP-Polypeptid zur Verwendung nach einem der Ansprüche 1 oder 3-9 oder Verwendung nach einem der Ansprüche 2-9, wobei das SAP-Polypeptid eine Aminosäuresequenz umfasst, die mindestens zu 99% identisch zur SEQ ID NO: 1 ist.

14. SAP-Polypeptid zur Verwendung nach einem der Ansprüche 1 oder 3-9 oder Verwendung nach einem der Ansprüche 2-9, wobei das SAP-Polypeptid fünf Polypeptide der SEQ ID NO:1 umfasst.

## Revendications

1. Polypeptide amyloïde P sérique (APS) pour une utilisation dans le traitement, la prévention, ou la réduction de la sévérité d'une mucosite ou d'un trouble lié à une mucosite chez un patient.

2. Utilisation d'un polypeptide APS dans la préparation d'un médicament pour le traitement, la prévention, ou la réduction de la sévérité d'une mucosite ou d'un trouble lié à une mucosite chez un patient.

3. Polypeptide APS pour une utilisation selon la revendication 1, ou utilisation selon la revendication 2, dans lequel la mucosite ou le trouble lié à une mucosite est choisi parmi une mucosite gastro-intestinale, une mucosite orale, une mucosite alimentaire, une mucosite oesophagienne, des ulcères gastriques, des ulcères duodénaux, des érosions de l'estomac et de l'oesophage, une colite, un reflux gastro-oesophagien, et une maladie inflammatoire chronique de l'intestin.

4. Polypeptide APS pour une utilisation selon la revendication 1 ou 3, ou utilisation selon l'une des revendications 2 ou 3, dans lequel la mucosite est associée à une radiothérapie, une chimiothérapie, ou une combinaison de ceux-ci.

5. Polypeptide APS pour une utilisation selon l'une quelconque des revendications 1, 3 ou 4, ou utilisation selon l'une quelconque des revendications 2 à 4, dans lequel le patient présente un risque de développer une mucosite.

6. Polypeptide APS pour une utilisation selon la revendication 5, ou utilisation selon la revendication 5, dans lequel le polypeptide APS est pour une administration avant, simultanée, ou après un traitement qui fait courir un risque au patient de développer une mucosite.

7. Polypeptide APS pour une utilisation selon la revendication 6, ou utilisation selon la revendication 6, dans lequel le traitement qui fait courir un risque au patient de développer une mucosite comprend une radiothérapie, une chimiothérapie, ou une combinaison de ceux-ci.

8. Polypeptide APS pour une utilisation selon la revendication 6 ou 7, ou utilisation selon la revendication 6 ou 7, dans lequel le polypeptide APS est pour le traitement d'une mucosite ou d'un trouble lié à une mucosite chez un patient étant traité pour un cancer.

9. Polypeptide APS pour une utilisation selon la revendication 8, ou utilisation selon la revendication 8, dans lequel le cancer est choisi parmi un cancer de la tête, un cancer du cou, un cancer des seins, un cancer des poumons, un cancer des ovaires, un cancer de la prostate, un lymphome, une leucémie, ou un cancer gastro-intestinal.

10. Polypeptide APS pour une utilisation selon l'une quelconque des revendications 1 ou 3 à 9, ou utilisation selon l'une quelconque des revendications 2 à 9, dans lequel le polypeptide APS comprend une séquence d'acides aminés qui est au moins 95% identique à SEQ ID NO : 1.

11. Utilisation selon la revendication 2, dans laquelle le médicament comprend en outre un facteur de croissance choisi parmi la vélafermine et la palifermine.

12. Polypeptide APS pour une utilisation selon l'une quelconque des revendications 1 ou 3 à 9, ou utilisation selon l'une quelconque des revendications 2 à 9, dans lequel le polypeptide APS comprend une séquence d'acides aminés qui est au moins 97% identique à SEQ ID NO : 1.

13. Polypeptide APS pour une utilisation selon l'une quelconque des revendications 1 ou 3 à 9, ou utilisation selon l'une quelconque des revendications 2 à 9, dans lequel le polypeptide APS comprend une séquence d'acides aminés qui est au moins 99% identique à SEQ ID NO : 1.

14. Polypeptide APS pour une utilisation selon l'une quelconque des revendications 1 ou 3 à 9, ou utilisation selon l'une quelconque des revendications 2 à 9, dans lequel le polypeptide APS comprend cinq polypeptides de SEQ ID NO : 1.
